# EUROPEAN PATENT SPECIFICATION

(11) **EP 2 526 919 B1**
(45) Date of publication and mention of the grant of the patent: **05.10.2016**
(21) Application number: 12180626.9
(22) Date of filing: 24.05.2007
(51) Int. Cl.: A61J 1/20

(54) **Reconstitution device**
Rekonstitutionsvorrichtung
Dispositif de reconstitution

(30) Priority: 25.05.2006 US 803187 P
(43) Date of publication of application: 28.11.2012
(62) Divisional of application: 07762316.3
(73) Proprietor: Bayer Healthcare LLC, Whippany, NJ 07981-0915 (US)
(72) Inventor: Tuckwell, Jonathan David, Cambridge CB4 1YB, Cambridgeshire (GB); Dyer, Robert, Cambridge 02138, MA Massachusetts (US); Kivlin, Robert Owen, Norfolk PE31 6BH, Norfolk (GB); Palmer-Felgate, John Paul, Winchester SO22 4JY, Hampshire (GB); Avery, Matthew Burgess, Palo Alto, CA 94306 (US); Skinner, Kevin, George, Oakland, CA 94619-3727 (US); Kadamus, Chris, Jamaica Plain, MA 02130, MA Massachusetts (US); Wood, Lee, South Wales CF31 4QP, South Wales (GB); Schwan, Peter, 51373 Leverkusen (DE); Arlett, Ben, Cambridge, MA 02139, MA Massachusetts (US)
(74) Representative: BIP Patents

(56) References cited:
- FR-A- 2 753 624

## Description

### Priority

This application claims the benefit of U.S. Provisional Application No. 60/803,187, filed May 25, 2006.

### Field of the Invention

The present application relates generally to reconstitution devices. More particularly, the application relates to an improved reconstitution device for connecting a closed receptacle and a container, such as a syringe.

### Description of Related Art

In the domain of drug-packaging, it is known to store a component of a medicinal preparation, such as for example its active ingredient, in a recipient closed by a stopper of relatively non-rigid material, for example of elastomer. A liquid may be introduced into this recipient after perforation of the stopper in order to dissolve the component contained in the recipient or place it in suspension, with a view to obtaining a medicinal preparation in liquid form ready to be administered to the patient.

Traditional devices include a base adapted to cover the neck of the recipient and extending in a flange forming an inner bore while a plunger is adapted to slide in the bore, between a position disengaged with respect to the stopper and an engaged position in which a hollow needle borne by the plunger traverses this stopper. The displacement of the plunger from its disengaged position towards its engaged position is effected manually by an operator.

However, many traditional devices do not include an actuating mechanism to prevent unwanted use of the device and to facilitate user interaction. Since the reconstitution device is not meant to be reused, unwanted actuation of the device could be wasteful and incur unnecessary costs. Furthermore, many traditional devices do not prevent the device from being reused.

FR2753624 A1 discloses a connecting device between a first receptacle and a second receptacle. The device comprises an apparatus for perforating a stopper, including a faucet and a filtering chamber isolated from outside by a filter. The device further comprises an element for displacing with guidance the perforating apparatus, an element for fastening the skirt on the neck, a plunger mounted in the internal bore on which the perforating apparatus is fixed, for sliding by simple pressure, and an element for definitively stopping the plunger when the end of the perforating apparatus has completely traversed the stopper. One or more clipping members, belonging to the plunger are constituted by teeth and are distributed about the axis of the plunger. The clipping members are elastic in order to be returned in centrifugal or centripetal manner. One or more complementary stop members, disposed on the skirt, consisting for example of an annular flank, against which or under which the teeth are blocked when the plunger is fully displaced towards the stopper. The device disclosed in FR2753624 A1 does not prohibit that the perforating apparatus after partially penetrating the stopper and is being retracted again.

Accordingly, it is desirable to develop a reconstitution device that facilitates user interaction by increasing the likelihood that the user follows the proper steps in the reconstitution process, as well as sufficiently preventing the device from being inadvertently actuated or reused.

### SUMMARY

The present invention is directed to a reconstitution device as claimed in the independent claim 1 and the claims dependent thereon.

The present invention helps to solve the shortcomings of the prior art by facilitating user interaction by increasing the likelihood that the user follows the proper steps in the reconstitution process. The device further provides an improved means of tamper-proofing than currently available reconstitution devices.

These as well as other aspects and advantages will become apparent to those of ordinary skill in the art by reading the following detailed description, with reference where appropriate to the accompanying drawings. Further, it should be understood that the embodiments described in this summary and elsewhere are intended to illustrate the invention by way of example only.

### BRIEF DESCRIPTION OF THE DRAWINGS

Exemplary embodiments of the invention are described herein with reference to the drawings, in which:
Figure 1 is an expanded view of a reconstitution device which is not subject-matter of the invention claimed herein;
Figure 2 is an expanded view of the plunger shown in Figure 1;
Figure 3 is a cross-sectional view of the reconstitution device of Figure 1;
Figure 4 is a perspective view of the locking mechanism of the device of Figure 1;
Figures 5A - 5C are perspective views of the actuation of the reconstitution device of Figure 1;
Figure 6 is a perspective view of a portion of the reconstitution device of Figure 1;
Figure 7 is a perspective view of the reconstitution device of Figure 1 including a top;
Figures 8A and 8B are perspective views of a second receptacle in use with the reconstitution device of Figure 1;
Figure 9 is a cross-sectional view of an embodiment of a reconstitution device of the present invention;
Figure 10 is a cross-sectional view of the reconstitution device of Figure 9 including a ratcheting mechanism; and
Figure 11 is a perspective view of the reconstitution device of Figure 9 including a top.

### DETAILED DESCRIPTION

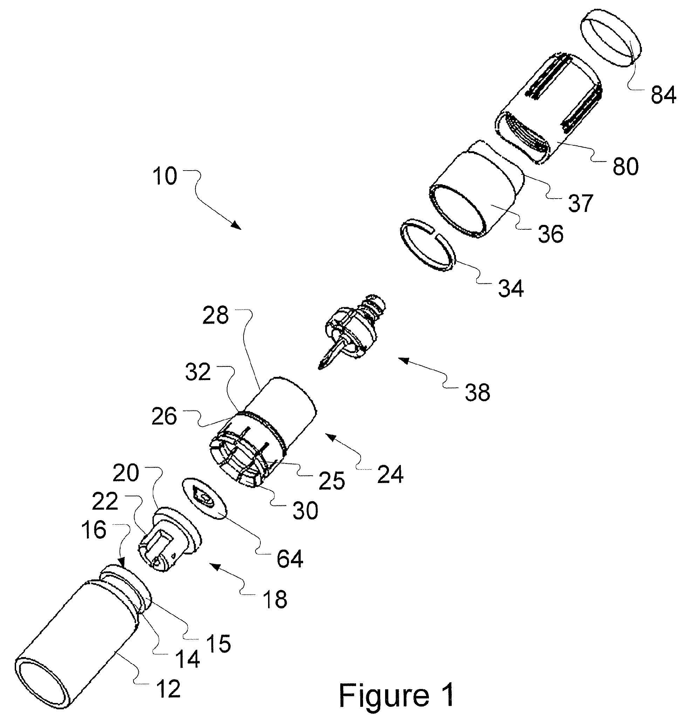

Figure 1 depicts a reconstitution device 10 which is not subject-matter of the invention claimed herein. The device 10 includes a receptacle 12 for storing a first component of a pharmaceutical preparation (not shown), such as its active ingredient, for example. The receptacle 12 includes an opening 16 surrounded or partially surrounded by a neck 14. The neck 14 also includes a lip 15. The opening 16 in the neck 14 allows for a second component (not shown), such as a liquid, to be introduced into the receptacle 12 and mix with the first component. A stopper 18 is positioned in the opening 16 of the neck 14 to block access to the receptacle 12. The stopper 18 is made of a relatively non-rigid material, such as elastomer. The stopper 18 includes a top portion 20 located against the lip 15, and a bottom portion 22 located within the opening of the neck 14. The top portion 20 is capable of being perforated, thereby allowing access to the receptacle 12.

The device 10 further includes a cap 24 secured to the neck 14 of the receptacle 12. The cap 24 has a first end 26, which is secured to the neck 14 of the receptacle 12, and a second end 28 located opposite the first end 26. The first end 26 of the cap 24 surrounds at least a portion of the stopper 18. The first end 26 of the cap 24 includes one or more flexible legs 30 so the cap 24 can expand to fit over the lip 15 of the neck 14 during the manufacturing process, and then contract to securely mate with the neck 14. The cap 24 further includes a step portion 32 located between the first end 26 and the second end 28, thereby separating the first end 26 from the second end 28. The first end 26 of the cap 24 has a larger diameter than the second end 28 of the cap 24.

The first end 26 of the cap 24 is secured to the receptacle 12 by a C-clip 34, which is positioned in an indentation 25 in the first end 26 of the cap 24. Therefore, the C-clip 34 is not located directly on the neck 14 of the receptacle 12 and no scratching occurs. The C-clip 34 is made of a material not subject to heat degradation, such as metal for example, so that the device 10 is resistant to heat tampering.

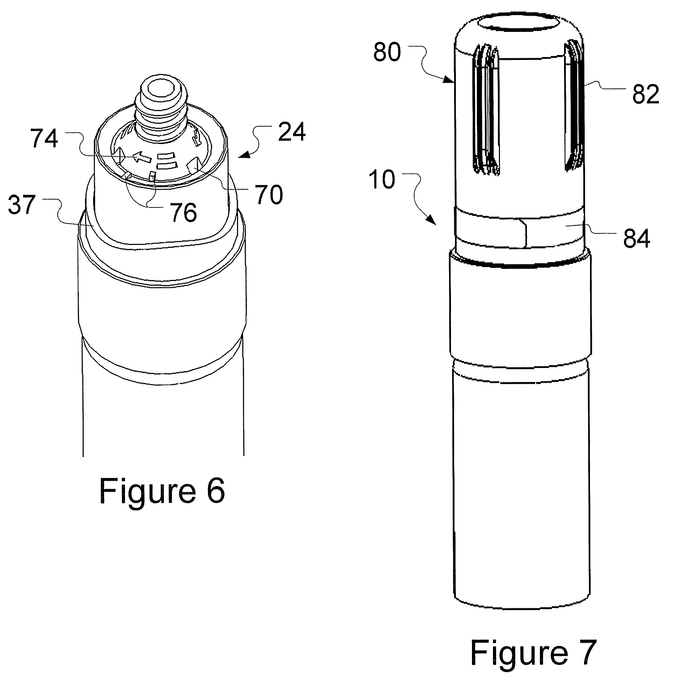

The first end 26 of the cap is surrounded by a sleeve 36 for protection. The sleeve 36 is prevented from sliding off the cap 24 by the step portion 32. The sleeve 36 includes a wave-shaped part 37 to facilitate opening by a user (not shown), as can be seen in detail in Figure 6. The sleeve 36 further includes a textured surface to facilitate gripping of the device 10 by a user.

Assembly of the first end 26 of the cap 24 to the receptacle 12 is carried out using various methods. For example, an assembly method includes initially inserting the C-clip 34 into the indentation 25, and then sliding the cap 24 over the stopper 18 and the lip 15. As another example, an assembly method includes initially placing the C-clip 34 onto the second end 28 of the cap 24, then sliding the sleeve 36 over the second end 28 in such a way that the sleeve 36 contacts the C-clip 34 and pushes the C-clip 34 into the indentation 25, and then sliding the cap 24 over the stopper 18 and the lip 15. In accordance with these exemplary methods, as the cap 24 is slid over the stopper 18 and the lip 15, the opening in the C-clip 34 allows the flexible legs 30 to expand as the flexible legs 30 pass over the lip 15, and the flexible legs 30 contract after passing over the lip 15 to secure the cap to the receptacle 12.

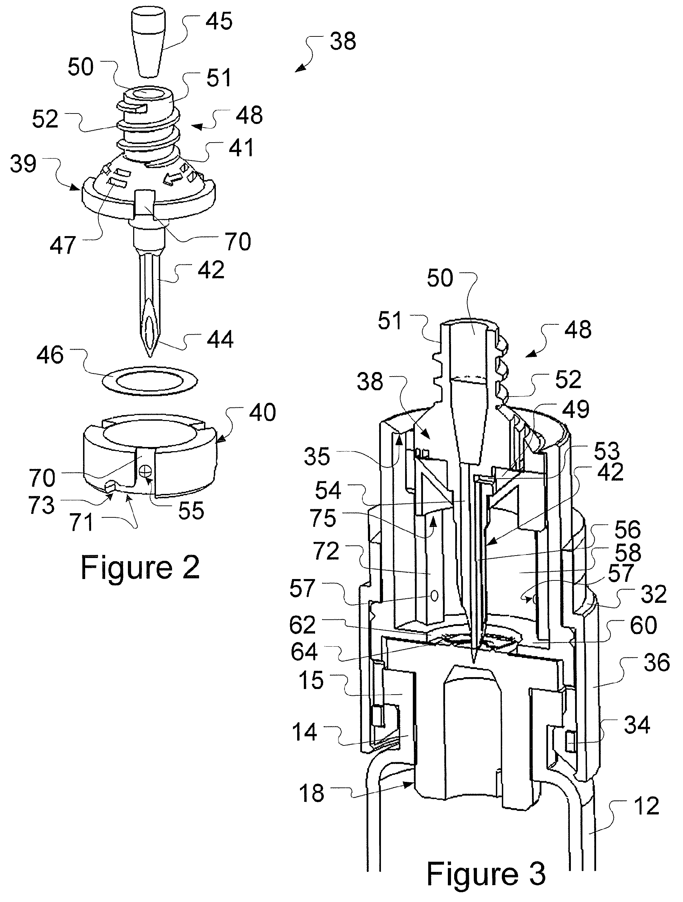

Figure 2 depicts details of a plunger 38 located at the second end 28 of the cap 24. The cap 24 also includes a ridge 35, shown in Figure 3, which is used to secure the plunger 38 within the cap 24. The plunger 38 comprises a first portion 39. The first portion 39 of the plunger 38 includes a top surface 41. The top surface 41 may be arranged in various configurations. For example, the top surface 41 may be dome-shaped (i.e., a domed top surface). As another example, the top surface 41 may be angled (i.e., an angled top surface). Other exemplary configurations of the top surface 41 are also possible.

The first portion 39 of the plunger 38 further includes a shaft 42 extending downwardly in a direction towards the stopper 18. The shaft 42 includes a pointed end 44 for piercing the top portion 20 of the stopper 18, thereby allowing the shaft 42 access to the receptacle 12. The pointed end 44 is angled to cooperate with a locking mechanism 64, which is described in detail below.

In one embodiment, the shaft 42 is elliptical-shaped (e.g., oval shaped). The shaft 42 being elliptical-shaped requires less applied force when the shaft 42 pierces the stopper 18. In alternative embodiments, the shaft 42 has a cylindrical shape or a rectangular shape.

The plunger 38 comprises a second portion 40 which is located below the first portion 39. The second portion 40 of the plunger 38 preferably has the same shape as the first portion 39, (e.g., an elliptical shape, a cylindrical shape, or a rectangular shape).

In one exemplary embodiment, the first portion 39 of the plunger 38 is permanently attached to the second portion 40 of the plunger 38 so as to prevent leaks (e.g., a fluid leak) between where the first portion 39 mates to the second portion 40. Various methods may be used to permanently attach the first portion 39 of the plunger 38 to the second portion 40 of the plunger 38. For example, the first portion 39 of the plunger 38 is permanently attached to the second portion 40 of the plunger 38 by ultrasonic welding. As another example, the first portion 39 of the plunger 38 is permanently attached to the second portion 40 of the plunger 38 by use of an adhesive (e.g., a glue). Other exemplary methods for permanently attaching the first portion 39 of the plunger 38 to the second portion 40 of the plunger 38 are also possible.

The plunger 38 may further include one or more filtering mechanisms. For example, a fluid filter 45 may be located in the first portion 39 of the plunger so as to filter any liquid that is introduced into the plunger 38. Furthermore, an air filter 46 may be located in a cavity 49 (shown in Figure 3) between the first portion 39 of the plunger 38 and the second portion 40 of the plunger 38 so as to filter any air that is introduced into the receptacle 12. The air filter 46 may be made from any of a variety of materials, but is preferably made of polyethersulphone (PES). Both filters may be located in line with the shaft 42 of the plunger 38.

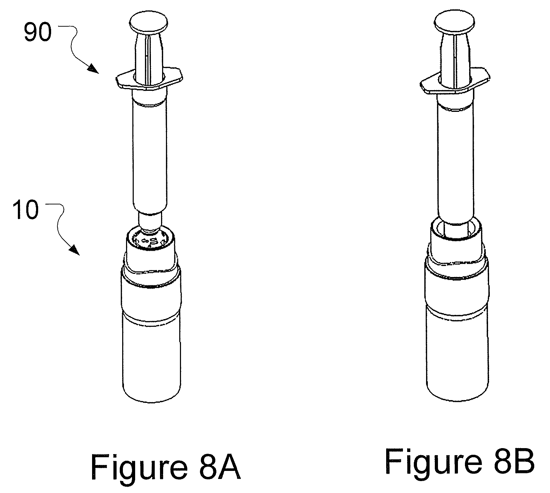

The first portion 39 of the plunger 38 may include a male element 48 extending from the top surface 41 in an upward direction opposite the shaft 42. The male element 48 is configured to receive a second receptacle 90, such as a syringe, for example, as shown in Figures 8A and 8B. The male element 48 includes an inner bore 50 and an outer surface 51. The outer surface 51 includes a thread 52 for mating with the second receptacle 90.

An advantage of the dome or angled shape of the top surface 41 is that the male element 48 visibly extends above the top surface 41. When the male element 48 receives the second receptacle 90 (e.g., by fastening a threaded syringe to the thread 52 of the male element 48), a user can clearly see when the second receptacle 90 is not in contact with the top surface 41. In this way, a user fastening the second receptacle 90 can continue to turn the second receptacle 90 and clearly tell when the second receptacle 90 makes contact with the top surface 41. Thus, the user knows when second receptacle 90 is fully secured to the male element 48.

Referring to Figure 3, the shaft 42 of the plunger 38 includes a first longitudinal channel 54. The first longitudinal channel 54 establishes communication between the receptacle 12 and the inner bore 50 of the male element 48. The first channel 54 allows fluid to pass through the shaft 42 of the plunger 38 and into the receptacle 12.

The shaft 42 of the plunger 38 includes a second longitudinal channel 56. The end of the second channel 56 located opposite the pointed end 44 of the shaft 42 interfaces to an air channel return 53. The air channel return 53 interfaces to a cavity 49. The cavity 49 is formed by joining the first portion 39 of the plunger 38 to the second portion 40 of the plunger 38. The second channel 56 functions as an air path to allow air to travel out of the receptacle 12 through the shaft 42 of the plunger 38, into the air channel return 53, through the filter 46, and then through the vent 47 located on the top surface 41 of the plunger 38. The air exiting the vent 47 is vented to the atmosphere surrounding the device 10.

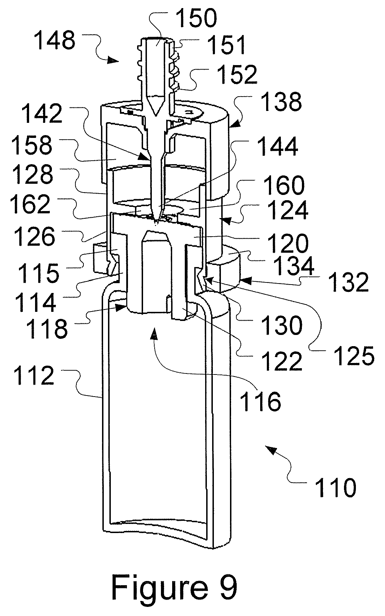

The cap 24 further includes an inner bore 58 having an annular plate 60 with an aperture 62. The aperture 62 is coaxially aligned with the shaft 42 of the plunger 38. The plunger 38 may be configured to slide along the inner bore 58 of the cap 24, or alternatively, the plunger 38 may be configured to slide along an outer surface of the cap, as shown in Figure 9, which will be described below. When the plunger 38 is pushed downward toward the receptacle 12, the shaft 42 moves downwardly through the aperture 62 to pierce the top portion 20 of the stopper 18.

The device 10 may include a locking mechanism 64 for preventing upward movement of the plunger 38 after downward movement of the plunger 38 has occurred. In this manner, the device 10 is prevented from multiple uses by restraining the plunger 38 in an engaged position, that is, when the shaft 42 pierces the stopper 18.

The locking mechanism 64 uses St. Venant's principle, which states deformations due to stress concentrations are not observed at a distance of three major diameters from the stress concentration. In accordance with this principle, if the locking mechanism 64 is made of a thin material that deforms an area of the shaft 42 greater than the thickness of the thin material as the shaft 42 is moved in a downward direction, then the locking mechanism 64 will prevent the shaft 42 from moving in an upward direction because the locking mechanism will fall in the deformed area of the shaft 42.

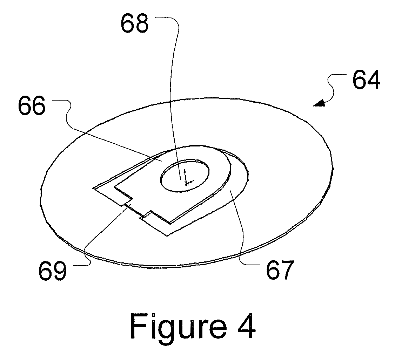

As shown in Figure 4, the locking mechanism 64 may include a thin, cylindrically shaped material. Alternatively, the locking mechanism 64 could be another shape such as elliptical or rectangular. The locking mechanism 64 may be made of any flexible material, such as metal or plastic, for example.

The locking mechanism 64 may be located at various locations within the device 10. For example, as shown in Figure 3, the locking mechanism 64 may be located below the annular plate 60. As another example, the locking mechanism 64 may be located above the annular plate 60. As yet another example, the locking mechanism 64 could be incorporated into the cap 24 (e.g., the locking mechanism could be made of the same material as the cap 24 and molded to the cap 24).

The locking mechanism 64 includes a tongue 66 extending therefrom, and being located within a first aperture 67 of the locking mechanism 64. The tongue 66 is connected to the first aperture 67 at tab 69, and therefore the tongue 66 is capable of flexing in the vertical direction. The tongue 66 includes a second aperture 68 that is coaxially aligned with the shaft 42. The diameter of the second aperture 68 is slightly larger than the diameter of the shaft 42. The tongue 66 is bent upward, as shown in Figure 4.

In operation, the locking mechanism 64 functions as a unidirectional cam. When the plunger 38 is pushed downwardly, the shaft 42 presses the tongue 66 downwardly, thereby opening the second aperture 68 enough to allow the shaft 42 to pass through the second aperture 68 and pierce the stopper 18. The locking mechanism 64 is triggered when a user attempts to pull the plunger 38 upwardly after the plunger 38 has been pushed downwardly.

When the plunger 38 is pulled upwardly, the shaft 42 pulls upwardly on the tongue 66 which causes the tongue 66 to flex vertically in an upward direction. The flexing causes the second aperture 68 to change shape from a first shape (*e.g*., a cylindrical shape when the second aperture 68 is substantially perpendicular to the shaft 42) to a second shape (*e.g*., an elliptical shape when the second aperture 68 is in a position not perpendicular to the shaft 42). In this way, the shape of the second aperture 68 is different from the shape of the shaft 42. Since the shaft 42 cannot freely pass through the second aperture 68 due in part to their different shapes, the tongue 66 then cuts into the shaft 42, creating a stress concentration, or notch (not shown), in the shaft 42. The second aperture 68 in the tongue 66 fits into the notch and prevents the plunger 38 from upward movement. Therefore, the plunger 38 may not be removed from the stopper 18, or moved in an upward direction, without damaging the device 10.

The locking mechanism 64 ensures a smooth downward motion of the plunger 38 with low actuation force and prevents reuse of the device 10 by retaining the plunger 38 in a downward position due to a high retaining force (relative to the actuation force). The locking mechanism 64 prevents return motion at any point during the downward movement of the shaft 42. Thus, the locking mechanism 64 improves upon retaining clips used in other reconstitution devices to secure a plunger only after the plunger has reached the most downward point of the plunger's downward travel.

The device 10 includes an actuating mechanism. The actuating mechanism includes at least one indentation 70 located on an outer circumference of the plunger 38, on both the first portion 39 and the second portion 40, and at least one protrusion 72 located on the inner bore 58 of the cap 24, on the second end 28 of the cap 24. The indentation 70 located on the second portion 40 includes a passage 71 and a groove 73.

When the device 10 is in a disengaged position, the at least one indentation 70 and the at least one protrusion 72 are not aligned and the plunger 38 is prevented from downward movement. Thus, the actuating mechanism acts as a safety from accidentally pushing down on the plunger 38 and piercing the stopper 18. An upper end 75 of the at least one protrusion 72 is rounded so that the upper end 75 of the at least one protrusion 72 rests in the groove 73 when the device 10 is in the disengaged position.

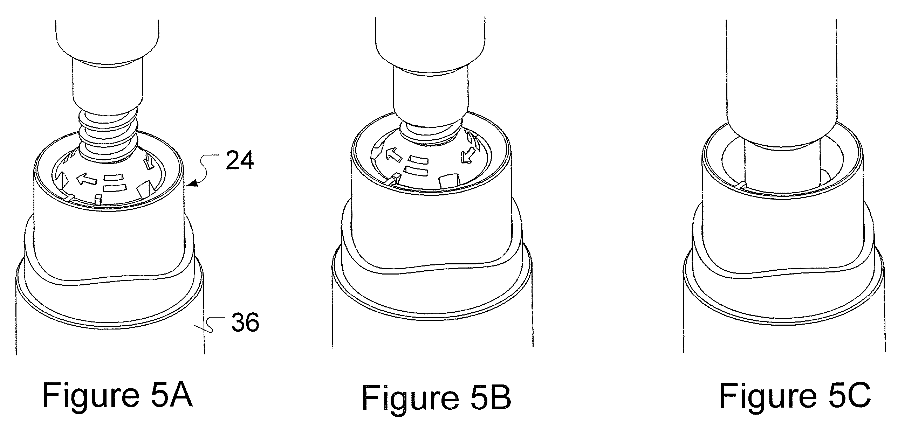

In order to use the device 10, a user rotates the plunger 38 a given number of degrees until the at least one indentation 70 aligns with the at least one protrusion 72, as shown in Figures 5A and 5B. The top surface 41 of the plunger 38 may include direction markers 74 to indicate to a user which direction to turn the plunger, as best seen in Figure 6. The top surface 41 may further include alignment markers 76 to indicate to the user when the actuating mechanism is aligned.

As the user rotates the plunger 38, the at least one protrusion 72 also moves downward so that the at least one protrusion 72 can move from the groove 73 to the passage 71 and then move from the passage 71 to the at least one indentation 70. The movement from the passage 71 to the at least one indentation 70 can provide tactile feedback and/or audible feedback to the user so that the user knows when the plunger 38 has been rotated the given number of degrees and it is acceptable to push the plunger 38 in a downward direction.

The given number of degrees the plunger 38 is rotated to align the at least one indentation 70 with the at least one protrusion 72 depends on various factors. For example, the factors include: (i) the number of indentations of the at least one indentation 70 and the number of protrusions of the at least one protrusion 72, (ii) the spacing between each protrusion (if more than one protrusion is used), and the spacing between each indentation (if more than one indentation is used), (iii) the position of the at least one indentation 70 relative to the position of the at least one protrusion 72 at the time the device 10 is assembled and/or at the time the plunger 38 is to be rotated, and (iv) the size of the at least one indentation 70 and the size of the at least one protrusion 72 (*e.g*., each protrusion and indentation are 1/36^{th} (i.e., 10 degrees) of the circumference of the inner bore 58 of the cap 24 and the outer circumference of the plunger 38, respectively).

In one exemplary embodiment, the at least one protrusion 72 includes three protrusions substantially equally spaced around the inner bore 58 of the cap (*e.g*., substantially spaced one hundred twenty degrees apart), and the at least one indentation 70 includes three indentations substantially equally spaced around the outer circumference of the plunger 38 (*e.g*., substantially spaced one hundred twenty degrees apart). In accordance with this exemplary embodiment, the given number of degrees the plunger 38 is rotated to align the at least one indentation 70 with the at least one protrusion 72 is preferably is about thirty (30) degrees. However, depending on one or more of the factors described above, the given number of degrees are substantially between five (5) degrees and one hundred twenty (120) degrees.

Referring to Figure 5C, the plunger 38 is then pushed downwardly into the engaged position so the shaft 42 passes through the aperture 62 in the cap 24 and pierces the stopper 18. Once the shaft 42 has pierced the stopper 18, the locking mechanism 64 prevents the plunger 38 from being pulled back upwardly to the disengaged position.

The device 10 may include a feedback mechanism that provides a user of the device 10 with feedback regarding operation of the device 10. For example, the feedback indicates that the shaft 42 has traveled an optimum distance into the stopper 18. Alternatively, or in combination, the feedback indicates that the plunger 38 has traveled an optimum distance within the cap 24 and that the user should not push the plunger 38 any further. Other examples of the feedback provided by the feedback mechanism are also possible.

The feedback mechanism may be arranged in various configurations. For example, as shown in Figures 2 and 3, the feedback mechanism may comprise (i) a convex bump 55 located on the surface of the second portion 40 of the plunger 38 and extending into the indentation 70, and (ii) a convex bump 57 on the protrusion 72.

The convex bump 55 is molded as part of the second portion 40. The convex bump 55 extends 0.2 mm to 1.0 mm (preferably 0.3 mm to 0.6 mm) away from the second portion 40. A widest portion of the convex bump 55 has a diameter between 0.2 mm to 1.0 mm (preferably 0.3 mm to 0.6 mm).

The convex bump 57 is be molded as part of the indentation 70. The convex bump 57 extends 0.2 mm to 1.0 mm (preferably 0.3 mm to 0.6 mm) away from the indentation 70. A widest portion of the convex bump 57 has a diameter between 0.2 mm to 1.0 mm (preferably 0.3 mm to 0.6 mm).

In operation, and by way of example, as the plunger 38 travels in a direction towards the stopper 18, the feedback mechanism provides tactile feedback as the convex bump 55 on the indentation 70 travels past the convex bump 57 on the protrusion 72. The tactile feedback may be felt by the user. Moreover, as the convex bump 55 on the indentation 70 travels past the convex bump 57 on the protrusion 72, a sound may be made such that the feedback mechanism also provides audible feedback.

With reference to Figure 7, the device 10 includes a top 80 which is positioned over the second end 28 of the cap 24 and the plunger 38. The top 80 protects the plunger 38. The top 80 further includes a textured surface 82 for facilitating gripping and removal of the top 80 by a user. Additionally, a tamper-proof mechanism 84 is be located on the device 10 to indicate to a user whether the device 10 has been used. The tamper-proof mechanism 84 comprises any type of indicator, such as a seal, a holographic label, or a tab, for example.

In operation, the device 10 is in the disengaged position, that is, when the protrusions 72 of the cap 24 and the indentations 70 of the plunger 38 are not aligned, and the shaft 42 of the plunger 38 is not piercing the stopper 18, as shown in Figure 8A. Once the top 80 is removed from the device 10 by a user, the tamper-proof mechanism 84 will be broken. A user may then attach a second receptacle 90, such as a syringe, to the receptacle 12. To activate the device 10, a user then rotates the plunger 38 about the given number of degrees (*e.g*., 30 degrees) so the indentations 70 on the plunger 38 align with the protrusions 72 on the cap 24. The plunger 38 then is pushed in a downward direction toward the stopper 18 into the engaged position, as shown in Figure 8B. The shaft 42 of the plunger 38 pierces the stopper 18, allowing access to the opening 16 of the receptacle 12. The contents of the second receptacle 90 may then be introduced into the receptacle 12 to mix with the component. The mixed contents may then be pulled back into the second receptacle 90. A needle (not shown) may then be secured to the second receptacle 90, and the complete and active drug may be administered to a patient.

Referring to Figure 9, an embodiment 110 of the reconstitution device of the present invention is shown. The device 110 includes a receptacle 112 for storing a first component of a pharmaceutical preparation (not shown), such as its active ingredient, for example. The receptacle 112 includes an opening 116 surrounded or partially surrounded by a neck 114. The neck 114 may also include a lip 115. The opening 116 in the neck 114 allows for a second component, such as a liquid (not shown), to be introduced into the receptacle 112 and mix with the first component. A stopper 118 is positioned in the opening 116 of the neck 114 to block access to the receptacle 112. The stopper 118 may be made of a relatively non-rigid material, such as elastomer. The stopper 118 includes a top portion 120 located against the lip 115, and a bottom portion 122 located within the opening 116 of the neck 114. The top portion 120 is capable of being perforated, to allow access to the receptacle 112.

The device 110 further includes a cap 124 secured to the neck 114 of the receptacle 112. The cap 124 has a first end 126, which is secured to the neck 114 of the receptacle 112, and a second end 128 located opposite the first end 126. The first end 126 of the cap 124 surrounds at least a portion of the stopper 118. The first end 126 of the cap 124 includes a protrusion 130 for securing the first end 126 to the lip 115 of the receptacle 112.

The first end 126 of the cap 124 is further secured to the receptacle 112 by a crimp ring 132, which is positioned in an indentation 125 in the first end 126 of the cap 124. The crimp ring 132 extends completely or around only a portion of the circumference of the first end 126 of the cap 124. The crimp ring 132 may be made of metal or a polymer with low creep sensitivity. The crimp ring 132 further includes an upper surface 134 for attaching to a top 180, which will be described below.

The device 110 includes a plunger 138 located at the second end 128 of the cap 124. The plunger 138 includes a shaft 142 extending in a direction towards the stopper 118. The shaft 142 includes a pointed end 144 for piercing the top portion 120 of the stopper 118, thereby allowing the shaft 142 access to the receptacle 112. The shaft 142 may further include a filtering mechanism (not shown) similar in structure and function to the filtering mechanism disclosed above with respect to device 10.

The plunger 138 may also include a male element 148 extending in a direction opposite the shaft 142. The male element 148 is configured to receive a second receptacle (not shown), such as a syringe, for example. The male element 148 includes an inner bore 150 and an outer surface 151. The outer surface 151 includes a thread 152 for mating with the second receptacle.

The shaft 142 of the plunger 138 also include first and second longitudinal channels (not shown) which establish communication between the receptacle 112 and the inner bore 150 of the male element 148. The longitudinal channels are similar in structure and function to the longitudinal channels described above with respect to device 10. The plunger 138 is further configured to slide along the outer circumference of the cap 124, as shown in Figure 9.

The cap 124 further includes an inner bore 158 having an annular plate 160 with an aperture 162. The aperture 162 is coaxially aligned with the shaft 142 of the plunger 138. When the plunger 138 moves downward toward the receptacle 112, the shaft 142 moves downwardly through the aperture 162 to pierce the top portion 120 of the stopper 118.

The device 110 includes a mechanism that prevents upward movement of the plunger 138 after downward movement of the plunger 138 towards the stopper 118 has occurred.

The device 110 includes a ratcheting mechanism that prevents upward movement of the plunger 138 after downward movement of the plunger 138 towards the stopper 118 has occurred. The ratcheting mechanism is used to retain the plunger to the device 110.

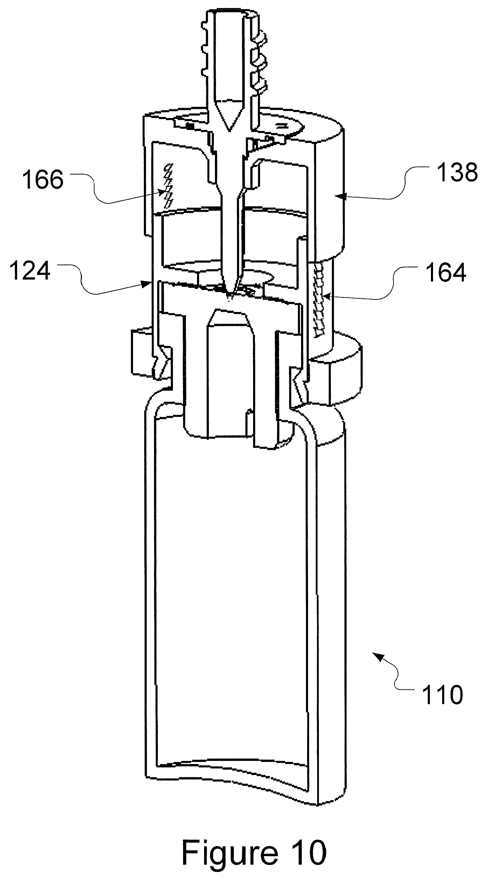

The ratcheting mechanism includes one or more series of ribs and one or more series of teeth. Each of the one or more series of ribs corresponds to one of the series of teeth. Figure 10 shows the device 110 including (i) a series of ribs 164 located on an outer surface of the cap 124, and (ii) a series of teeth 166 located on an inner surface of the plunger 138. The series of ribs 164 corresponds to another set of teeth (not shown) located on the inner surface of the plunger 138. The series of teeth 166 corresponds to another series of ribs (not shown) located on the outer surface of the cap 124.

In this way, as the plunger 138 is moved in a downward direction towards the stopper 118, one or more teeth of a series of teeth (not shown) travel over the series of ribs 164, whereas if upward movement of the plunger 138 is attempted, one or more teeth of the series of teeth (not shown) encounter the series of ribs 164 so as to prevent the one or more teeth as well as the plunger 138 from moving upwards. Similarly, as the plunger 138 is moved in a downward direction towards the stopper 118, one or more teeth of a series of teeth 166 travel over a series of ribs (not shown), whereas if upward movement of the plunger 138 is attempted, one or more teeth of the series of teeth 166 encounter the series of ribs (not shown) so as to prevent the series of teeth 166 as well as the plunger 138 from moving upwards.

The cap 124 and plunger 138 of the device 110 further includes an actuating mechanism similar in structure and function to the actuating mechanism described above with respect to the device 10.

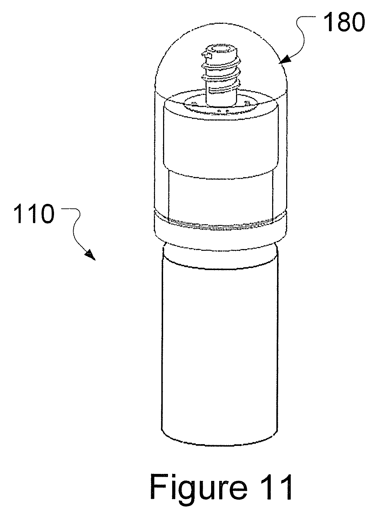

The device 110 may include a top 180, as shown in Figure 11, which fits over the second end 128 of the cap 124 and the plunger 138, and attaches to the crimp ring 132. The top 180 protects the device 110.

Moreover, the top 180 and the crimp ring 132 may be formed as a single piece (*i.e*., a top and crimp ring combination (not shown)). To accommodate the top and crimp ring combination, the cap 124 has a first end and a second end, and the first end is larger in diameter than the second end (similar to the first end 26 of the cap 24 and the second end 28 of the cap 24 shown in Figure 1). In this way, the top and crimp ring combination slides over the plunger 138 so as to allow the cap 124 to be secured to the receptacle 112. To use the device 110 with the top and crimp ring combination, the top is broken off (*e.g*., by twisting the top) and the crimp ring continues to secure the cap 124 to the receptacle 112.
Additionally, a tamper-proof mechanism (not shown) may be located on the device 110 to indicate to a user whether the device has been used. The tamper-proof mechanism comprises any type of indicator, such as a seal, a holographic label, or a tab, for example.

In operation, the device 110 is in a disengaged position, that is, the shaft 142 of the plunger 138 is not piercing the stopper. Once the top 180 is removed from the device 110, the tamper-proof mechanism will be broken. A user may then attach a second receptacle, such as a syringe, to the receptacle 112. The plunger 138 may then be pushed in a downward direction toward the stopper 118 into an engaged position. The shaft 142 of the plunger 138 pierces the stopper 118, allowing access to the opening 116 of the receptacle 112. The contents of the second receptacle may then be introduced into the receptacle 112 to mix with the component. The mixed contents may then be pulled back into the second receptacle. A needle (not shown) may then be secured to the second receptacle, and the complete and active drug may be administered to a patient.

While certain features and embodiments of the present invention have been described in detail herein, it is to be understood that the invention encompasses all modifications and enhancements within the scope of the following claims.

## Claims

1. A reconstitution device (110) comprising:
a receptacle (112) having an opening (116) surrounded by a neck (114);
a cap (124) including a first end (126), a second end (128), and an inner bore (158) having an aperture (162), the first end (126) being secured to the receptacle (112);
a stopper (118) located within the opening (116) of the neck (114), the stopper (118) including a portion capable of being perforated;
a plunger (138) secured to the second end (128) of the cap (124), the plunger (138) having a shaft (142); and
an actuating mechanism located on the plunger (138) and the cap (124);
wherein when the actuating mechanism is activated, the plunger (138) travels downwardly to perforate the stopper (118),
**characterized in** the plunger being adapted to slide along the outer circumference of the cap (124) and in
the reconstitution device further comprising a ratcheting mechanism that prevents upward movement of the plunger (138) after downward movement of the plunger (138) towards the stopper (118) has occurred,
wherein the ratcheting mechanism comprises one or more series of ribs (164) and one or more series of teeth (166), and
wherein each series of ribs (164) corresponds to one of the series of teeth (166).

2. The device (110) of claim 1,
wherein the actuating mechanism comprises at least one protrusion on the cap (124) and at least one indentation on the plunger (138),
wherein the plunger (138) is rotatable for aligning the at least one protrusion with the at least one indentation, and
wherein the plunger (138) is movable in a downward direction when the at least one protrusion is aligned with the at least one indentation.

3. The device (110) of claim 1,
wherein the one or more series of ribs (164) are located on an outer surface of the cap (124), and
wherein the one or more series of teeth (166) are located on an inner surface of the plunger (138).

4. The device (110) of claim 1, wherein the ratcheting mechanism retains the plunger (138) to the device (110).

5. The device (110) of claim 1, wherein the first end (126) of the cap (124) includes a protrusion (130) for securing the first end (126) of the cap (124) to a lip (115) of the receptacle (112).

6. The device (110) of claim 1,
wherein the cap (124) includes an inner bore (158) having an annular plate (160) with an aperture (162),
wherein the aperture (162) is coaxially aligned with the shaft (142) of the plunger (138), and
wherein, when the plunger (138) moves downward toward the receptacle (112), the shaft (142) of the plunger (138) moves downwardly through the aperture (162) to pierce a top portion (120) of the stopper (118).

7. The device (110) of any of claims 1 to 6,
wherein the plunger (138) includes a male element (148) that extends in a direction opposite the shaft (142),
wherein the male element (148) is configured to receive a second receptacle,
wherein the male element includes an inner bore (150) and an outer surface (151),
wherein the outer surface (151) includes a thread (152) for mating with the second receptacle.

8. The device (110) of claim 7, wherein the second receptacle is a syringe.

9. The device (110) of claim 7, wherein contents of the second receptacle can be introduced into the receptacle (112) after the shaft (142) of the plunger (138) pierces the stopper (118) to allow access to the opening (116) of the receptacle (112).

10. The device (110) of claim 7, wherein the shaft (142) of the plunger (138) includes a first longitudinal channel and a second longitudinal channel to establish communication between the receptacle (112) and the inner bore (150) of the male element (148).

11. The device (110) of any of claims 1 to 10, further comprising:
a crimp ring (132),
wherein the first end (126) of the cap (124) includes an indentation (125), and
wherein the crimp ring (132) is positioned in the indentation (125).

12. The device (110) of claim 11, further comprising:
a top (180) that fits over the second end (128) of the cap (124) and the plunger (138),
wherein the top (180) attaches to the crimp ring (132).

## Patentansprüche

1. Rekonstitutionsvorrichtung (110), umfassend:
einen Aufnahmebehälter (112) mit einer Öffnung (116), die von einem Hals (114) umgeben ist;
eine Kappe (124) mit einem ersten Ende (126), einem zweiten Ende (128) und einer Innenbohrung (158) mit einer Öffnung (162), wobei das erste Ende (126) an dem Aufnahmebehälter (112) befestigt ist;
einen Stopfen (118), der in der Öffnung (116) des Halses (114) angeordnet ist, wobei der Stopfen (118) einen Abschnitt aufweist, der perforierbar ist;
einen Kolben (138), der an dem zweiten Ende (128) der Kappe (124) befestigt ist, wobei der Kolben (138) einen Schaft (142) aufweist; und
einen Betätigungsmechanismus, der auf dem Kolben (138) und der Kappe (124) angeordnet ist;
wobei sich der Kolben (138) bei Aktivierung des Betätigungsmechanismus nach unten bewegt, um den Stopfen (118) zu perforieren,
**dadurch gekennzeichnet, dass** der Kolben ausgelegt ist, entlang des Außenumfangs der Kappe (124) zu gleiten, und dass
die Rekonstitutionsvorrichtung ferner einen Rastmechanismus umfasst, der eine Aufwärtsbewegung des Kolbens (138) verhindert, nachdem eine Abwärtsbewegung des Kolbens (138) zum Stopfen (118) erfolgt ist,
wobei der Rastmechanismus eine oder mehrere Reihen von Rippen (164) und eine oder mehrere Reihen von Zähnen (166) umfasst, und
wobei jede Reihe von Rippen (164) einer der Reihen von Zähnen (166) entspricht.

2. Vorrichtung (110) nach Anspruch 1,
wobei der Betätigungsmechanismus wenigstens einen Vorsprung auf der Kappe (124) und wenigstens eine Vertiefung auf dem Kolben (138) umfasst,
wobei der Kolben (138) zur Ausrichtung des wenigstens einen Vorsprungs mit der wenigstens einen Vertiefung drehbar ist, und
wobei der Kolben (138) in eine Abwärtsrichtung bewegbar ist, wenn der wenigstens eine Vorsprung mit der wenigstens einen Vertiefung ausgerichtet ist.

3. Vorrichtung (110) nach Anspruch 1,
wobei die eine oder mehreren Reihen von Rippen (164) auf einer Außenseite der Kappe (124) angeordnet sind, und
wobei die eine oder mehreren Reihen von Zähnen (166) auf einer Innenseite des Kolbens (138) angeordnet sind.

4. Vorrichtung (110) nach Anspruch 1, wobei der Rastmechanismus den Kolben (138) an der Vorrichtung (110) hält.

5. Vorrichtung (110) nach Anspruch 1, wobei das erste Ende (126) der Kappe (124) einen Vorsprung (130) zur Befestigung des ersten Endes (126) der Kappe (124) an einer Lippe (115) des Aufnahmebehälters (112) aufweist.

6. Vorrichtung (110) nach Anspruch 1,
wobei die Kappe (124) eine Innenbohrung (158) mit einer ringförmigen Platte (160) mit einer Öffnung (162) aufweist,
wobei die Öffnung (162) koaxial mit dem Schaft (142) des Kolbens (138) ausgerichtet ist, und
wobei sich der Schaft (142) des Kolbens (138) bei der Abwärtsbewegung des Kolbens (138) zum Aufnahmegefäß (112) nach unten durch die Öffnung (162) bewegt, um einen oberen Abschnitt (120) des Stopfens (118) zu durchstechen.

7. Vorrichtung (110) nach einem der Ansprüche 1 bis 6,
wobei der Kolben (138) ein männliches Element (148) aufweist, das sich in eine Richtung gegenüber dem Schaft (142) erstreckt,
wobei das männliche Element (148) zur Aufnahme eines zweiten Aufnahmegefäßes ausgelegt ist,
wobei das männliche Element eine Innenbohrung (150) und eine Außenseite (151) aufweist,
wobei die Außenseite (151) ein Gewinde (152) aufweist, das mit dem zweiten Aufnahmegefäß zusammenpasst.

8. Vorrichtung (110) nach Anspruch 7, wobei das zweite Aufnahmegefäß eine Spritze ist.

9. Vorrichtung (110) nach Anspruch 7, wobei der Inhalt des zweiten Aufnahmegefäßes in das Aufnahmegefäß (112) eingeführt werden kann, nachdem der Schaft (142) des Kolbens (138) den Stopfen (118) durchstochen hat, um Zugang zur Öffnung (116) des Aufnahmegefäßes (112) zu gestatten.

10. Vorrichtung (110) nach Anspruch 7, wobei der Schaft (142) des Kolbens (138) einen ersten langgestreckten Kanal und einen zweiten langgestreckten Kanal aufweist, um eine Verbindung zwischen dem Aufnahmegefäß (112) und der Innenbohrung (150) des männlichen Elements (148) herzustellen.

11. Vorrichtung (110) nach einem der Ansprüche 1 bis 10, ferner umfassend:
einen Crimp-Ring (132),
wobei das erste Ende (126) der Kappe (124) eine Vertiefung (125) aufweist, und
wobei der Crimp-Ring (132) in der Vertiefung (125) positioniert ist.

12. Vorrichtung (110) nach Anspruch 11, ferner umfassend:
eine Oberseite (180), die über das zweite Ende (128) der Kappe (124) und den Kolben (138) passt,
wobei die Oberseite (180) am Crimp-Ring (132) befestigt wird.

## Revendications

1. Dispositif de reconstitution (110) comprenant :
un réceptacle (112) comportant une ouverture (116) entourée par un col (114) ;
un capuchon (124) comprenant une première extrémité (126), une seconde extrémité (128) et un alésage intérieur (158) comportant une ouverture (162), la première extrémité (126) étant fixée au réceptacle (112) ;
un bouchon (118) situé à l'intérieur de l'ouverture (116) du col (114), le bouchon (118), comprenant une partie pouvant être perforée ;
un piston (138) solidement fixé à la seconde extrémité (128) du capuchon (124), le piston (138) comportant une tige (142) ; et
un mécanisme d'actionnement situé sur le piston (138) et le capuchon (124),
dans lequel, quand on active le mécanisme d'actionnement, le piston (138) se déplace vers le bas pour perforer le bouchon (118),
**caractérisé en ce que** le piston est apte à coulisser le long de la circonférence extérieure du capuchon (124) et **en ce que** le dispositif de reconstitution comprend en outre un mécanisme à cliquet qui empêche un mouvement ascendant du piston (138) après que se soit produit un mouvement descendant du piston (138) vers le bouchon (118),
dans lequel le mécanisme à cliquet comprend une ou plusieurs séries de nervures (164) et une ou plusieurs séries de dents (166), et
dans lequel chaque série de nervures (164) correspond à une des séries de dents (166).

2. Dispositif (110) selon la revendication 1,
dans lequel le mécanisme d'actionnement comprend au moins une partie saillante sur le capuchon (124) et au moins une indentation sur le piston (138),
dans lequel on peut faire tourner le piston (138) pour aligner ladite partie saillante avec ladite indentation, et
dans lequel on peut déplacer le piston (138) vers le bas quand ladite partie saillante est alignée avec ladite indentation.

3. Dispositif (110) selon la revendication 1,
dans lequel lesdites séries de nervures (164) sont situées sur une surface extérieure du capuchon (124), et
dans lequel lesdites séries de dents (166) sont situées sur une surface intérieure du piston (138).

4. Dispositif (110) selon la revendication 1, dans lequel le mécanisme à cliquet retient le piston (138) au dispositif (110).

5. Dispositif (110) selon la revendication 1, dans lequel la première extrémité (126) du capuchon (124) comprend une partie saillante (130) pour solidement fixer la première extrémité (126) du capuchon (124) à une lèvre (115) du réceptacle (112).

6. Dispositif (110) selon la revendication 1,
dans lequel le capuchon (124) comprend un alésage intérieur (158) comportant une plaque annulaire (160) ayant une ouverture (162),
dans lequel l'ouverture (162) est alignée coaxialement avec la tige (142) du piston (138), et
dans lequel, quand le piston (138) se déplace vers le bas vers le réceptacle (112), la tige (142) du piston (138) se déplace vers le bas par l'ouverture (162) pour percer une partie supérieure (120) du bouchon (118).

7. Dispositif (110) selon l'une quelconque des revendications 1 à 6,
dans lequel le piston (138) comprend un élément mâle (148) qui s'étend dans une direction opposée à celle de la tige (142),
dans lequel l'élément mâle (148) est configuré pour recevoir un second réceptacle,
dans lequel l'élément mâle comprend un alésage intérieur (150) et une surface extérieure (151),
dans lequel la surface extérieure (151) comprend un filetage (152) pour s'accoupler avec le second réceptacle.

8. Dispositif (110) selon la revendication 7, dans lequel le second réceptacle est une seringue.

9. Dispositif (110) selon la revendication 7, dans lequel le contenu du second réceptacle peut être introduit dans le réceptacle (112) après que la tige (142) du piston (138) a percé le bouchon (118) pour permettre d'accéder à l'ouverture (116) du réceptacle (112).

10. Dispositif (110) selon la revendication 7, dans lequel la tige (142) du piston (138) comprend un premier canal longitudinal et un second canal longitudinal pour établir la communication entre le réceptacle (112) et l'alésage intérieur (150) de l'élément mâle (148).

11. Dispositif (110) selon l'une quelconque des revendications 1 à 10, comprenant en outre :
une bague de sertissage (132),
dans lequel la première extrémité (126) du capuchon (124) comprend une indentation (125), et
dans lequel la bague de sertissage (132) est mise en place dans l'indentation (125).

12. Dispositif (110) selon la revendication 11, comprenant en outre :
un couvercle (180) qui s'adapte sur la seconde extrémité (128) du capuchon (124) et le piston (138), dans lequel le couvercle (180) se fixe à la bague de sertissage (132).
